# EUROPEAN PATENT APPLICATION

(11) **EP 3 047 789 A1**
(43) Date of publication of application: **27.07.2016**
(21) Application number: 15746057.7
(22) Date of filing: 02.02.2015
(51) Int. Cl.: A61B 1/04, G02B 23/24

(54) **ELECTRONIC ENDOSCOPIC SYSTEM, ELECTRONIC ENDOSCOPE, POWER SUPPLY DEVICE, METHOD FOR OPERATING ELECTRONIC ENDOSCOPIC SYSTEM**

(30) Priority: 05.02.2014 JP 2014020751
(71) Applicant: Olympus Corporation, Hachioji-shi Tokyo 192-8507 (JP)
(72) Inventor: TABUCHI, Koichiro, Tokyo (JP)
(74) Representative: Gunzelmann, Rainer
(86) International application number: PCT/JP2015/052818
(87) International publication number: WO 2015/119070

(57) **Abstract**

The invention provides an electronic endoscope system in which an endoscope control section (21) of an electronic endoscope (2) generates a power requesting signal indicating an amount of power to be supplied to the electronic endoscope (2) in accordance with an operating state of an in-scope electric circuit (22) and transmits the power requesting signal to a video processor (1), and a power supply control section (11) of the video processor (1) causes a power variable section (12) and a variable regulator (13) to output power with the amount of power indicated by the received power requesting signal to the electronic endoscope (2).

## Description

### Technical Field

The present invention relates to an electronic endoscope system that supplies power from a power supply apparatus to an electronic endoscope, the electronic endoscope, the power supply apparatus and a method for operating the electronic endoscope system.

### Background Art

Conventionally, electronic endoscopes (video scope: hereinafter, abbreviated as "scope" as appropriate) are used which pick up and acquire an image of a subject by an image pickup device. This electronic endoscope is connected to a video processor that processes the acquired image (hereinafter abbreviated as "processor" as appropriate), receives a power supply from the video processor and operates, that is, the video processor also serves as a power supply apparatus for the electronic endoscope.

In such an electronic endoscope system that supplies power from the power supply apparatus to the electronic endoscope, when the electronic endoscope is connected to the power supply apparatus such as the video processor, information specific to the electronic endoscope such as information on a model indicating the type or the like of the electronic endoscope is sent from the electronic endoscope to the power supply apparatus. The power supply apparatus compares the received model with a database stored in the power supply apparatus, sets parameters such as a voltage value or current upper limit value (current limit) in accordance with the model, supplies power to the electronic endoscope and selects and executes an operating sequence in accordance with the model.

To be more specific, Japanese Patent Application Laid-Open Publication No. 2009-106343 describes a technique that provides a voltage detection section that detects an applied voltage value in a scope and controls the voltage applied to the scope based on the voltage value detected by the voltage detection section when a power supply circuit section of a processor supplies power to the scope.

Furthermore, Japanese Patent Application Laid-Open Publication No. 2009-201541 describes that in an image pickup system in which an electronic endoscope provided with a solid image pickup device is connected to a processor apparatus and a light source apparatus via a universal cord, a power supply circuit of the processor apparatus generates a supply voltage and a grounding voltage, and supplies those voltages to the solid image pickup device.

Furthermore, Japanese Patent Application Laid-Open Publication No. 2011-139795 describes an endoscope apparatus provided with an endoscope scope including an image pickup section, and an endoscope processor that performs image processing in which a voltage is applied to a peripheral circuit of the image pickup section from a variable voltage source of the endoscope processor.

According to the aforementioned conventional schemes, only a series of operations following an operation sequence selected in accordance with a model can be performed and a signal to be a trigger or the like provided in advance is transmitted at set timing. Therefore, depending on an operation state of the electronic endoscope, power is also supplied to a circuit which is functionally not necessary or a standby current flows thereto.

Furthermore, power consumption of the electronic endoscope varies depending on an operating state such as exposure operation, reading operation or an operating mode such as normal mode, high resolution mode or the like. In an electronic endoscope system provided with such an electronic endoscope, a higher voltage than a necessary voltage is supplied in advance so that even if a voltage drop occurs when power consumption increases so as to be able to secure a necessary voltage. However, if such an operation is performed, not only useless power is consumed but also power supplied in excess is converted to heat, causing the temperature in the electronic endoscope to increase.

The present invention has been implemented in view of the above-described circumstances, and it is an object of the present invention to provide an electronic endoscope system, an electronic endoscope, a power supply apparatus and a method for operating the electronic endoscope system capable of supplying necessary sufficient power to the electronic endoscope according to necessity.

### Disclosure of Invention

### Means for Solving the Problem

An electronic endoscope system according to an aspect of the present invention includes an electronic endoscope for observing an inside of a subject including an in-scope electric circuit, and a power requesting section that generates and transmits a power requesting signal indicating an amount of power to be supplied in accordance with an operating state of the in-scope electric circuit, and a power supply apparatus to which the electronic endoscope is connected including a power supply that supplies power to the electronic endoscope, a power supply control section that receives the power requesting signal and controls the power supply so as to output power with the amount of power indicated by the received power requesting signal to the electronic endoscope.

An electronic endoscope according to an aspect of the present invention is an electronic endoscope for observing an inside of a subject, the electronic endoscope including an in-scope electric circuit and a power requesting section that generates and transmits a power requesting signal indicating an amount of power to be supplied to the electronic endoscope in accordance with an operating state of the in-scope electric circuit.

A power supply apparatus according to an aspect of the present invention is a power supply apparatus to which an electronic endoscope for observing an inside of a subject is connected, the power supply apparatus including a power supply that supplies power to the electronic endoscope and a power supply control section that receives a power requesting signal indicating an amount of power to be supplied to the electronic endoscope, the power requesting signal being generated and transmitted by a power requesting section of the electronic endoscope in accordance with an operating state of an in-scope electric circuit of the electronic endoscope, and controls the power supply so as to output the power with the amount of power indicated by the received power requesting signal to the electronic endoscope.

A method for operating an electronic endoscope system according to an aspect of the present invention is a method for operating an electronic endoscope system in which an electronic endoscope and a power supply apparatus are connected, the method including a step of a power requesting section of the electronic endoscope generating and transmitting a power requesting signal indicating an amount of power to be supplied to the electronic endoscope in accordance with an operating state of an in-scope electric circuit of the electronic endoscope, a step of a power supply control section of the power supply apparatus receiving the power requesting signal and controlling a power supply of the power supply apparatus so as to output power with the amount of power indicated by the received power requesting signal to the electronic endoscope, and a step of the power supply supplying power to the electronic endoscope.

### Brief Description of the Drawings

Fig. 1 is a block diagram illustrating a configuration of an electronic endoscope system according to Embodiment 1 of the present invention;
Fig. 2 is a flowchart illustrating operation of the electronic endoscope system according to Embodiment 1 of the present invention;
Fig. 3 is a block diagram illustrating a configuration of an electronic endoscope system according to Embodiment 2 of the present invention;
Fig. 4 is a flowchart illustrating operation of the electronic endoscope system according to Embodiment 2 of the present invention;
Fig. 5 is a block diagram illustrating a configuration of an electronic endoscope system according to Embodiment 3 of the present invention; and
Fig. 6 is a flowchart illustrating operation of the electronic endoscope system according to Embodiment 3 of the present invention.

### Best Mode for Carrying Out the Invention

Hereinafter, embodiments of the present invention will be described with reference to the accompanying drawings.

### [Embodiment 1]

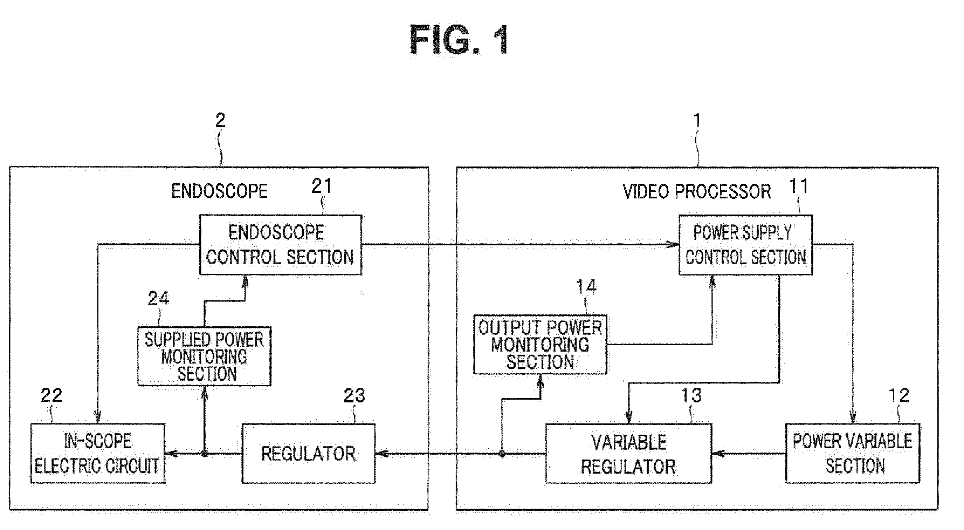

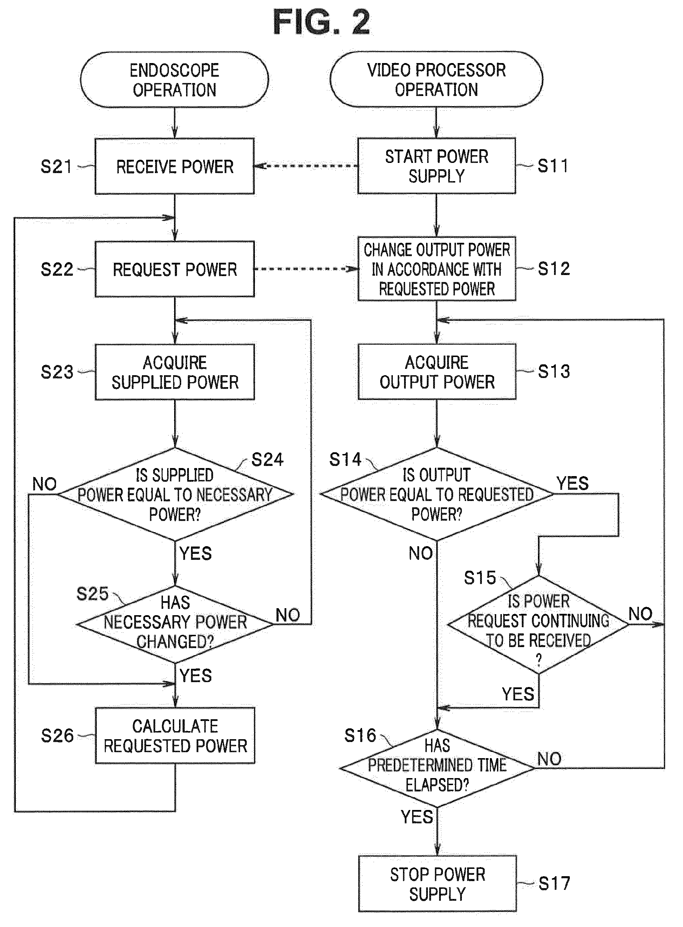

Fig. 1 and Fig. 2 illustrate Embodiment 1 of the present invention, and Fig. 1 shows a block diagram illustrating a configuration of an electronic endoscope system.

The electronic endoscope system is provided with a video processor 1 and an electronic endoscope 2 (hereinafter abbreviated as "endoscope 2" as appropriate).

With an endoscope 2 connected thereto, the video processor 1 processes an image signal obtained by the connected endoscope 2, and also serves as a power supply apparatus that supplies power to the endoscope 2 in the present embodiment. The video processor 1 is provided with a power supply control section 11, a power variable section 12 and a variable regulator 13 that constitute a power supply, and an output power monitoring section 14.

The endoscope 2 is a scope for observing an inside of a subject, actuated by power supplied from the video processor 1, picks up an image of the subject and transmits the picked-up image signal to the video processor 1. The endoscope 2 is provided with an endoscope control section 21, an in-scope electric circuit 22, a regulator 23 and a supplied power monitoring section 24.

The power supply control section 11 is constructed of, for example, an FPGA (field programmable gate array), receives a power requesting signal transmitted from the endoscope control section 21 which is a power requesting section and controls a power supply so as to output power with the amount of power indicated by the received power requesting signal to the endoscope 2.

The power supply control section 11 compares the amount of power indicated by the power requesting signal with the amount of output power detected by the output power monitoring section 14 and determines that the power supply to the endoscope 2 is abnormal, when the comparison result shows that the two amounts of power do not match even when a predetermined time elapses after receiving the power requesting signal and controlling the power supply or even when the comparison result shows that the two amounts of power match but if a time period during which the power requesting signal continues to be received from the endoscope 2 reaches a predetermined time.

The power variable section 12 is a variable resistor constructed of, for example, a digital potentiometer to change a voltage to be divided based on digital-like control from the power supply control section 11. The power variable section 12 then applies the divided voltage to the variable regulator 13.

The variable regulator 13 supplies power (voltage and current) stably, and is constructed of, for example, a regulator provided with, an ADJ terminal and a SHDN terminal, and the ADJ terminal changes the output voltage in accordance with the divided voltage applied from the power variable section 12.

The power variable section 12 and the variable regulator 13 constitute a power supply for supplying power to the endoscope 2.

The output power monitoring section 14 is constructed of, for example, an A/D converter and detects an amount of output power (voltage and current) outputted from the variable regulator 13 to the endoscope 2.

The power supplied from the variable regulator 13 of the video processor 1 is inputted to the regulator 23 of the endoscope 2. The regulator 23 stabilizes power supplied to the in-scope electric circuit 22.

The in-scope electric circuit 22 is provided with a circuit for picking up an image, specifically for example, provided with various circuits such as an image pickup device that converts an optical image of the subject to an image signal and an image pickup device drive circuit for driving the image pickup device configured on, for example, the electric circuit board.

The supplied power monitoring section 24 is constructed of, for example, an A/D converter and detects an amount of supplied power (voltage and current) supplied from the regulator 23 to the in-scope electric circuit 22.

The endoscope control section 21 is constructed of, for example, an FPGA (field programmable gate array) and controls the in-scope electric circuit 22 (e.g., control of an operating state such as exposure and reading).

The endoscope control section 21 functions as a power requesting section, generates a power requesting signal indicating an amount of power to be supplied to the endoscope 2 (in other words, an amount of power to be supplied by the video processor 1 which is a power supply apparatus to the endoscope 2) (hereinafter referred to as "amount of requested power" as appropriate) in accordance with an operating state of the in-scope electric circuit 22, and transmits the power requesting signal to the power supply control section 11 of the video processor 1.

To be more specific, the endoscope control section 21 calculates an amount of power to be supplied to the endoscope 2 (amount of requested power) based on an amount of required power required by the in-scope electric circuit 22 in accordance with the operating state of the in-scope electric circuit 22 and a circuit configuration of the endoscope 2 including the in-scope electric circuit 22 (specific examples includes a circuit configuration of the in-scope electric circuit 22 and the regulator 23).

The endoscope control section 21 in the present embodiment in particular, further calculates the amount of power to be supplied to the endoscope 2 more accurately based on the amount of supplied power detected by the supplied power monitoring section 24. More specifically, even when an amount of requested power calculated so as to obtain an amount of required power based on the circuit configuration of the endoscope 2 is requested from the video processor 1, an amount of requested power is corrected by multiplying the current amount of requested power by a coefficient (amount of required power/ amount of supplied power) when the actual supply amount of power is different from the amount of required power, and so on. By using the actual detection result in this way, it is possible to flexibly handle individual differences which may be generated even with an identical circuit configuration.

The power request by this endoscope control section 21 includes a request for a voltage and a request for a current, and the request for a current includes a request for a current limit value (upper limit value of an allowable current supply range). When there is a variation for each type (or each individual) in a current consumption value during operation, a current limit value is requested according to the variation.

The power request by the endoscope control section 21 starts immediately when a need arises and then continues to be made (is made consecutively) until the need is satisfied.

Next, Fig. 2 is a flowchart illustrating operation of the electronic endoscope system.

This processing starts when the endoscope 2 is connected to the video processor 1 and the video processor 1 supplies power necessary for the endoscope control section 21 to start operation (shown by a leftward dotted line arrow in Fig. 2) to the endoscope 2 (step S11).

Thus, the endoscope 2 receives a power supply from the video processor 1 and the endoscope control section 21 starts operation (step S21).

Next, the endoscope control section 21 generates a power requesting signal indicating an amount of required power assuming, as the amount of requested power, an amount of power necessary to start operation of the entire endoscope 2 immediately after the endoscope 2 is connected to the video processor 1 (this amount of power generally varies depending on the type (and by extension the model) of the endoscope 2) or an amount of power calculated in step S26 which will be described later if not immediately after and transmits the amount of requested power to the power supply control section 11 of the video processor 1 (shown by a rightward dotted line arrow in Fig. 2) (step S22).

The power supply control section 11 controls the power variable section 12 based on the amount of power (amount of requested power) indicated by the power requesting signal received from the endoscope control section 21. Thus, the power variable section 12 changes the amount of output power of the variable regulator 13 so as to correspond to the amount of requested power and the power with the changed amount of output power is immediately supplied to the endoscope 2 (step S12).

The supplied power monitoring section 24 always monitors the amount of power supplied from the regulator 23 to the in-scope electric circuit 22 and outputs the monitoring result to the endoscope control section 21 (step S23).

The endoscope control section 21 determines whether or not the amount of supplied power detected by the supplied power monitoring section 24 is equal to the amount of power (amount of required power) required by the in-scope electric circuit 22 in accordance with the operating state of the in-scope electric circuit 22 (step S24). Here, the amount of required power is set to a predetermined initial value immediately after the endoscope 2 is connected to the video processor 1 or set in step S26 which will be described later if not immediately after.

Here, if the amount of supplied power matches the amount of required power, the endoscope control section 21 further determines whether or not the amount of required power has changed because the operating state of the in-scope electric circuit 22 has changed or the like (step S25).

When it is determined that the amount of required power is not changed, the flow returns to step S23 and the amount of power supplied to the in-scope electric circuit 22 continues to be monitored.

When it is determined in step S24 that the amount of supplied power does not match the amount of required power or it is determined in step S25 that the amount of required power has changed, the endoscope control section 21 sets the amount of required power of the in-scope electric circuit 22 in accordance with the operating state of the in-scope electric circuit 22 and calculates the amount of power to be supplied to the endoscope 2 from the video processor 1 (amount of requested power) based on the set amount of required power and the circuit configuration of the in-scope electric circuit 22 and the regulator 23 (step S26). Note that although the amount of requested power can be calculated based on the amount of required power and the circuit configuration, as described above, in the present embodiment, the calculated amount of requested power may be more accurate using the amount of supplied power detected by the supplied power monitoring section 24. When the amount of requested power is calculated in this way, the flow returns to aforementioned step S22 and makes a power request.

On the other hand, the output power monitoring section 14 always monitors the amount of output power from the variable regulator 13 and the power supply control section 11 acquires this monitoring result (step S 13).

The power supply control section 11 then determines whether or not the amount of output power from the variable regulator 13 is equal to the amount of requested power from the endoscope control section 21 (step S 14).

Here, when it is determined that the amount of output power is equal to the amount of requested power, the power supply control section 11 determines whether or not the power requesting signal is continuing to be received from the endoscope 2 (step S15).

In this step S 15, when the power requesting signal is not continuing to be transmitted from the endoscope 2, this means that the endoscope 2 is receiving a required power supply normally, and therefore the flow returns to step S 13 and the power supply control section 11 continues to acquire the amount of output power.

On the other hand, in step S 14, when it is determined that the amount of output power is not equal to the amount of requested power or it is determined in step S15 that although the amount of output power is equal to the amount of requested power, the power requesting signal is continuing to be received, the power supply control section 11 determines whether or not a time period during which the amount of output power continues not to be equal to the amount of requested power or a time period during which a power requesting signal is continuing to be received has reached a predetermined time period (step S16).

In this step S16, when it is determined that the predetermined time has not elapsed, the flow returns to step S 13 and the power supply control section 11 continues to acquire the amount of output power.

On the other hand, when it is determined in step S16 that the predetermined time has elapsed, the power supply control section 11 determines that although control is being performed on the video processor 1 so as to supply the amount of requested power from the endoscope control section 21, the endoscope 2 is in an abnormal state in which it cannot receive the amount of requested power (e.g., a state in which wire breakage or increase in contact resistance occurs), and controls the power variable section 12 and variable regulator 13 to forcibly stop the power supply to the endoscope 2 (step S 17). In this case, it goes without saying that an announcement indicating the occurrence of an error or contents of an error (e.g., announcement to the user using display or voice) or the like may also be provided.

According to such Embodiment 1, since a power requesting signal indicating an amount of power to be supplied to the endoscope 2 is generated and transmitted in accordance with the operating state of the in-scope electric circuit 22, the video processor 1 needs only to output power with an amount of power indicated by the received power requesting signal, and it is thereby possible to achieve power saving without managing a complicated sequence. Therefore, it is possible to prevent heat generation in the endoscope 2 due to useless power consumption, thereby improve safety of operation and simplify the configuration of the video processor 1 and thereby achieve a cost reduction. It is further possible to flexibly follow a variation in characteristics for each individual which is not determined only by the model indicating the type or the like of the endoscope 2 or the amount of requested power in accordance with fine control during operation.

In this case, since the endoscope control section 21 calculates the amount of requested power based on the amount of required power required by the in-scope electric circuit 22 in accordance with the operating state and the circuit configuration of the endoscope 2, the video processor 1 need not consider the operating state and the circuit configuration of the endoscope 2 (that is, the video processor 1 need not know the circuit configuration of the endoscope 2, need not monitor the amount of power consumption of the endoscope 2 or need not calculate the amount of power to be outputted to the endoscope 2 based on the monitoring result), but only needs to output the requested power, and can reduce a processing load on the video processor 1.

Furthermore, the endoscope control section 21 can reduce useless power consumption with higher accuracy by calculating the amount of power to be supplied to the endoscope 2 more accurately based on the amount of supplied power detected by the supplied power monitoring section 24.

Thus, since the video processor 1 needs only to supply the requested power, the video processor 1 need not store a processing sequence, parameters or the like in advance in accordance with the model of the endoscope 2, can reduce the storage capacity and can also perform appropriate power control without supplying useless power even when an unknown new endoscope 2 is connected which is released after the video processor 1 is shipped.

Furthermore, although when the amount of power consumption of the endoscope 2 is monitored from the video processor 1, since this process is executed, for example, for every appropriate period, this causes lack of immediacy, with the configuration of the present embodiment, a power request is made at a point in time at which the endoscope 2 needs power, and therefore there is an advantage of high immediacy.

The video processor 1 which is a power supply apparatus is further provided with the output power monitoring section 14, the power supply control section 11 compares the amount of power requested from the endoscope 2 with the amount of output power detected by the output power monitoring section 14, and determines that the power supply to the endoscope 2 is abnormal when the comparison result does not show any match even after a predetermined time elapses, and can thereby apply safety measures such as stopping a power supply.

In addition, even when the amount of output power and the amount of requested power match if the time period during which the power requesting signal continues to be received reaches a predetermined time period, the power supply control section 11 determines that the power supply to the endoscope 2 is abnormal, and it is thereby possible to apply more stringent safety measures such as stopping the power supply. This can further improve safety as the electronic endoscope system.

According to the present embodiment, it is possible to supply necessary sufficient power to the endoscope 2 when a need arises.

### [Embodiment 2]

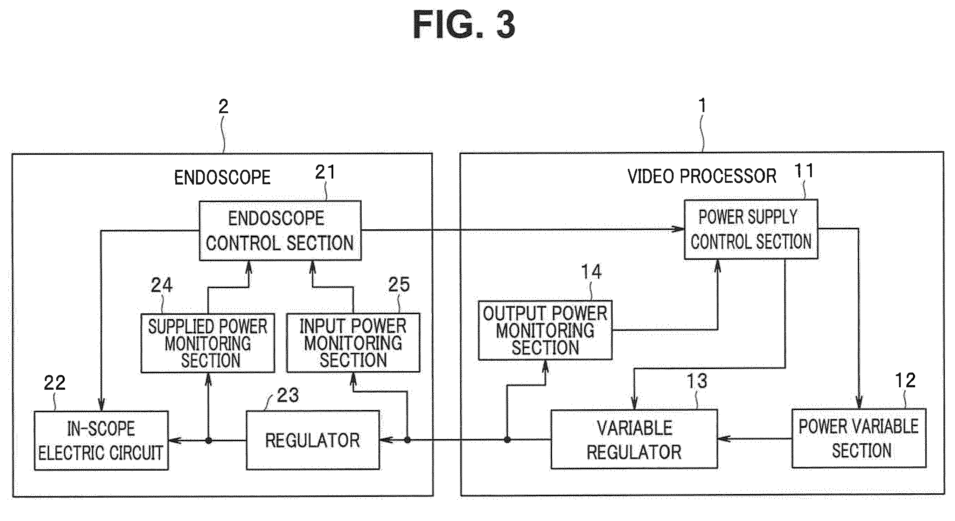

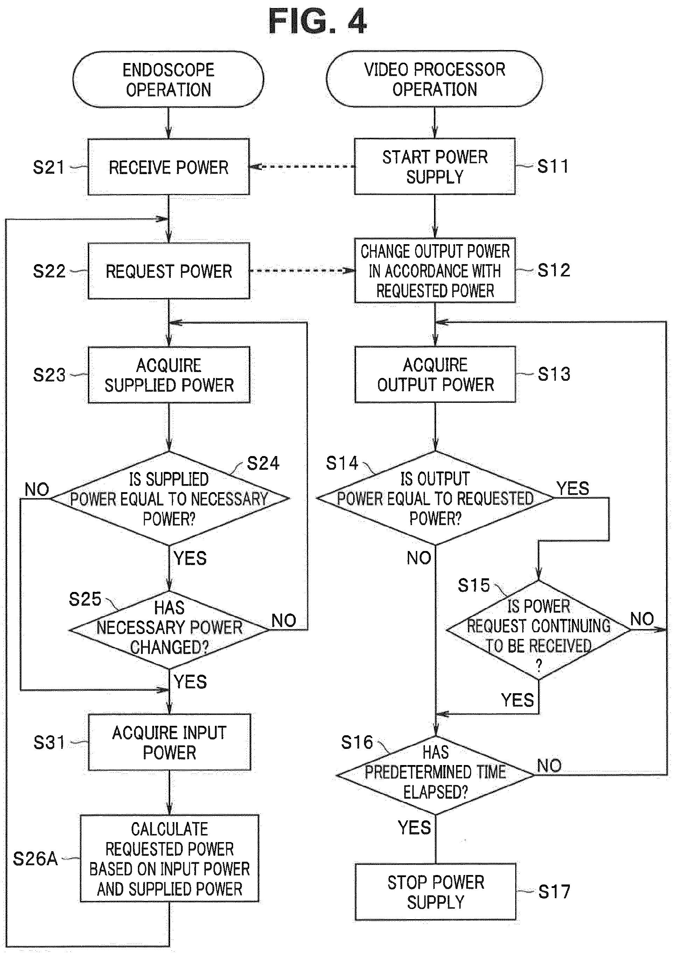

Fig. 3 and Fig. 4 illustrate Embodiment 2 of the present invention, Fig. 3 is a block diagram illustrating a configuration of an electronic endoscope system and Fig. 4 is a flowchart illustrating operation of the electronic endoscope system.

In Embodiment 2, parts similar to those in above Embodiment 1 are assigned the same reference numerals, description thereof is omitted as appropriate and only differences will be mainly described.

In addition to the configuration of the endoscope 2 of aforementioned Embodiment 1, the endoscope 2 of the present embodiment is further provided with an input power monitoring section 25 that detects an amount of input power inputted from the video processor 1 which is a power supply apparatus as shown in Fig. 3.

This input power monitoring section 25 is constructed of, for example, an A/D converter and detects the amount of input power (voltage and current) inputted from the video processor 1 to the regulator 23.

As shown in Fig. 4, when it is determined in step S24 that the amount of supplied power does not match the amount of required power or it is determined in step 25 that the amount of required power is changed, the endoscope control section 21 acquires an amount of input power detected by the input power monitoring section 25 (step S31).

Furthermore, the endoscope control section 21 that functions as a power requesting section calculates the amount of requested power to be supplied to the endoscope 2 much more accurately than in the aforementioned Embodiment 1 based not only on the amount of required power, the circuit configuration and the amount of supplied power but also based on the amount of input power detected by the input power monitoring section 25 (step S26A).

More specifically, as described in aforementioned Embodiment 1, the amount of requested power is corrected by multiplying it by a coefficient (amount of required power/amount of supplied power) after waiting for the amount of requested power to become equal to the amount of input power or the like.

Alternatively, when the amount of requested power does not become equal to the amount of input power after waiting for an appropriate predetermined time which is reasonable as a response time of power control, the coefficient by which the current amount of requested power is multiplied may be further modified into {(amount of required power/amount of supplied power)x(current amount of requested power/amount of input power)} to further refine the correction.

When the amount of requested power is calculated in this way, the flow returns to step S22 to make a power request.

Note that operation of the video processor 1 shown in Fig. 4 is basically the same as the operation of aforementioned Embodiment 1 shown in Fig. 2.

Such Embodiment 2 has substantially the same effects as those of aforementioned Embodiment 1, and when the endoscope control section 21 calculates an amount of requested power, monitoring results of both the amount of input power before the regulator 23 and the amount of supplied power after the regulator 23 are used, and it is thereby possible to grasp an effect of a voltage drop that occurs in the regulator 23 more accurately and transmit a more appropriate power requesting signal to the video processor 1.

This makes it possible to perform more accurate and more waste-free power control.

### [Embodiment 3]

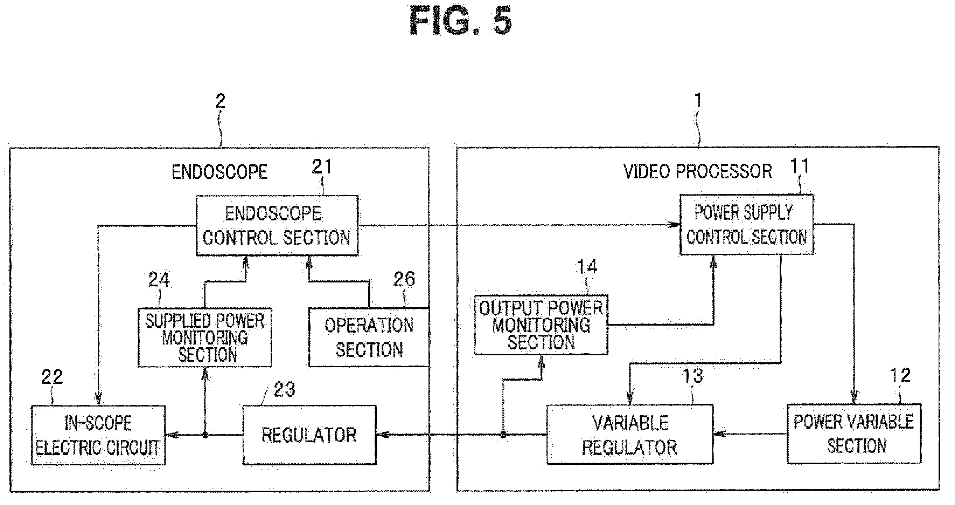

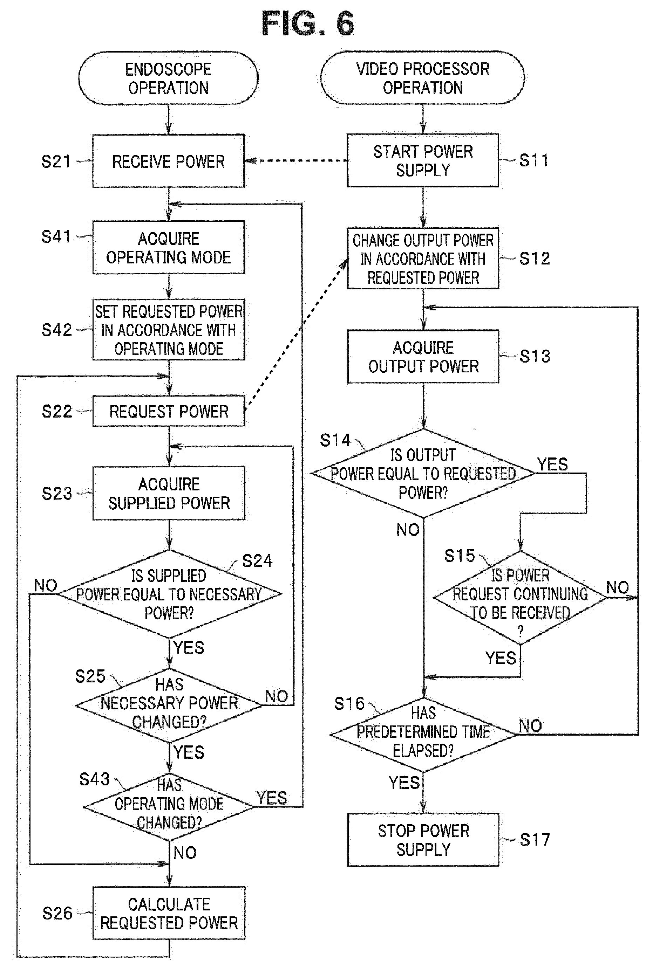

Fig. 5 and Fig. 6 illustrate Embodiment 3 of the present invention, Fig. 5 is a block diagram illustrating a configuration of an electronic endoscope system and Fig. 6 is a flowchart illustrating operation of the electronic endoscope system.

In Embodiment 3, parts similar to those in above Embodiments 1 and 2 are assigned the same reference numerals, description thereof is omitted as appropriate and only differences will be mainly described.

The electronic endoscope system of the present embodiment changes an amount of requested power according to an operating mode.

That is, in addition to the configuration of the endoscope 2 of aforementioned Embodiment 1, the endoscope 2 of the present embodiment is further provided with an operation section 26 including a mode changeover switch that can set any one of a plurality of operating modes having different power consumption levels as shown in Fig. 5. Note that Fig. 5 shows an example where the operation section 26 is added using the configuration of Embodiment 1 as the basis, but it goes without saying that the configuration of Embodiment 2 may be used as the basis.

Operating modes that can be set by this operation section 26 include a normal mode in which images are picked up at normal resolution and frame rate, a low power consumption mode in which at least one of the resolution and the frame rate is lowered compared to the normal mode (higher power-saving effect, however, can be obtained by lowering both of resolution and frame rate) and a high resolution mode in which images are picked up at maximum resolution (even if the frame rate cannot help but be lowered compared to the normal mode in order to cause the frame rate to fall within a range of an upper limit which exists in the rate of reading from the image pickup device), but the operating mode is not limited to these modes, and other operating modes may be naturally set. The normal mode is used for general image pickups, the low power consumption mode is used for, for example, when inserting the endoscope 2 before a region to be observed is reached and the high resolution mode is used for, for example, when a region to be observed is reached and more detailed still image is preferably obtained, but their applications are not limited to these.

When it is assumed that power consumption of the endoscope 2 when set in the high resolution mode is Ph, power consumption of the endoscope 2 when set in the normal mode is Pn, and power consumption of the endoscope 2 when set in the low power consumption mode is PI, magnitudes of power consumption in the respective modes are, for example, Ph>Pn>Pl.

Next, operation of the electronic endoscope system of the present embodiment is as shown in Fig. 6 and operation of the video processor 1 is basically the same as the operation of aforementioned Embodiment 1 shown in Fig. 2.

When the endoscope 2 receives power from the video processor 1 in step S21, the endoscope control section 21 that functions as a power requesting section acquires an operating mode currently set in the endoscope 2 (operating mode automatically set when power reception starts or operating mode set by the operation section 26) (step S41), and sets an amount of requested power to be supplied to the endoscope 2 in accordance with the acquired operating mode (step S42). Examples of the amount of requested power set in this step S42 include a requested voltage corresponding to the operating mode and a limit current corresponding to an operating mode.

After that, in step S22, a power requesting signal is generated and transmitted based on the set amount of requested power and further the process in step S23 is executed.

When it is determined in step S24 that the amount of supplied power matches the amount of required power and it is determined in step S25 that the amount of required power has not changed, it is further determined whether or not the operating mode has been changed by operation of the operation section 26 or the like (step S43).

When it is determined in this step S43 that the operating mode has changed, the flow returns to step S41 to acquire the changed operating mode and when it is determined that the operating mode has not changed, the process in step S26 is executed.

According to such Embodiment 3, substantially the same effects as those in aforementioned Embodiments 1 and 2 are obtained and a power requesting signal is generated and transmitted in accordance with the operating mode, and therefore only necessary power is supplied in the set operating mode and power-saving in accordance with the operating mode can be achieved.

Since the video processor 1 sets an appropriate current limit value in accordance with the operating mode in response to a request from the endoscope 2, the video processor 1 side need not store the current limit value in accordance with the model and the operating mode of the endoscope 2, can reduce the storage capacity, and even if an unknown new endoscope 2 is connected which is released after the video processor 1 is shipped and operated in an unknown operating mode, it is possible to perform appropriate power control.

Note that although the electronic endoscope system provided with an electronic endoscope and a power supply apparatus has been mainly described above, the present invention may also be a method for operating the electronic endoscope system as described above or a control program for causing a computer to control the electronic endoscope system as described above or a non-temporary and computer-readable recording medium that records the control program.

Moreover, the present invention is not limited to the aforementioned embodiments as they are, but can be implemented by modifying the components in an implementation phase without departing from the spirit and scope of range of the present invention. Furthermore, various aspects of the present invention can be formed in appropriate combinations of a plurality of components disclosed in the above embodiments. For example, several components may be deleted from the overall components shown in the embodiments. Furthermore, components among different embodiments may be combined as appropriate. It goes without saying that various modifications and applications can thus be made without departing from the spirit and scope of the present invention.

The present application claims priority based on Japanese Patent Application No. 2014-20751, filed on February 5, 2014, the disclosure of which is incorporated in the specification, the claims and the drawings of the present application by reference in its entirety.

## Claims

1. An electronic endoscope system comprising:
an electronic endoscope for observing an inside of a subject, the electronic endoscope including,
an in-scope electric circuit, and
a power requesting section that generates and transmits a power requesting signal indicating an amount of power to be supplied in accordance with an operating state of the in-scope electric circuit; and
a power supply apparatus to which the electronic endoscope is connected, the power supply apparatus including,
a power supply that supplies power to the electronic endoscope, and
a power supply control section that receives the power requesting signal and controls the power supply so as to output power with the amount of power indicated by the received power requesting signal to the electronic endoscope.

2. The electronic endoscope system according to claim 1, wherein the power requesting section calculates the amount of power to be supplied to the electronic endoscope based on an amount of required power required by the in-scope electric circuit in accordance with the operating state of the in-scope electric circuit and a circuit configuration of the electronic endoscope including the in-scope electric circuit.

3. The electronic endoscope system according to claim 2,
wherein the electronic endoscope further comprises a supplied power monitoring section that detects an amount of power supplied to the in-scope electric circuit, and
the power requesting section further calculates the amount of power to be supplied to the electronic endoscope based on the amount of supplied power detected by the supplied power monitoring section.

4. The electronic endoscope system according to claim 3,
wherein the electronic endoscope further comprises an input power monitoring section that detects an amount of input power inputted from the power supply apparatus, and
the power requesting section further calculates the amount of power to be supplied to the electronic endoscope based on the amount of input power detected by the input power monitoring section.

5. The electronic endoscope system according to claim 1,
wherein the electronic endoscope further comprises an operation section that can set any one of a plurality of operating modes with different power consumption levels, and
the power requesting section generates and transmits a power requesting signal indicating the amount of power to be supplied to the electronic endoscope in accordance with an operating mode set in the electronic endoscope.

6. The electronic endoscope system according to claim 1,
wherein the power supply apparatus further comprises an output power monitoring section that detects an amount of the output power outputted from the power supply, and
the power supply control section compares the amount of power indicated by the power requesting signal with the amount of output power detected by the output power monitoring section and determines that the power supply to the electronic endoscope is abnormal when a result of the comparison shows that both amounts of power do not match even when a predetermined time period elapses after receiving the power requesting signal and controlling the power supply.

7. The electronic endoscope system according to claim 6, wherein the power supply control section determines that the power supply to the electronic endoscope is abnormal even when the comparison result shows that both amounts of power match, if a time period during which the power requesting signal continues to be received reaches the predetermined time period.

8. An electronic endoscope for observing an inside of a subject, comprising:
an in-scope electric circuit; and
a power requesting section that generates and transmits a power requesting signal indicating an amount of power to be supplied to the electronic endoscope in accordance with an operating state of the in-scope electric circuit.

9. A power supply apparatus to which an electronic endoscope for observing an inside of a subject is connected, comprising:
a power supply that supplies power to the electronic endoscope; and
a power supply control section that receives a power requesting signal indicating an amount of power to be supplied to the electronic endoscope generated and transmitted by a power requesting section of the electronic endoscope in accordance with an operating state of an in-scope electric circuit of the electronic endoscope and controls the power supply so as to output the power with the amount of power indicated by the received power requesting signal to the electronic endoscope.

10. A method for operating an electronic endoscope system to which an electronic endoscope and a power supply apparatus are connected, the method comprising:
a step of a power requesting section of the electronic endoscope generating and transmitting a power requesting signal indicating an amount of power to be supplied to the electronic endoscope in accordance with an operating state of an in-scope electric circuit of the electronic endoscope;
a step of a power supply control section of the power supply apparatus receiving the power requesting signal and controlling a power supply of the power supply apparatus so as to output power with the amount of power indicated by the received power requesting signal to the electronic endoscope; and
a step of the power supply supplying power to the electronic endoscope.
